# EUROPÄISCHE PATENTSCHRIFT

(11) **EP 0 947 171 B1**
(45) Veröffentlichungstag und Bekanntmachung des Hinweises auf die Patenterteilung: **23.07.2003**
(21) Anmeldenummer: 99105859.5
(22) Anmeldetag: 23.03.1999
(51) Int. Cl.: A61B 17/22

(54) **Flexible Metallsonde zur Verwendung bei der intrakorporalen Stosswellen-Lithotripsie**
Flexible metal probe for use in intracorporal shock wave lithotripsy
Sonde flexible en métal utilisée pour la lithotripsie intracorporelle à ondes de chocs

(30) Priorität: 31.03.1998 DE 19814395
(43) Veröffentlichungstag der Anmeldung: 06.10.1999
(73) Patentinhaber: Ferton Holding S.A., 2800 Delemont (CH)
(72) Erfinder: Jerger, Thomas, 71065 Sindelfingen (DE); Horn, Uwe, 78467 Konstanz (DE); Goin, Emanuel, 1005 Lausanne (CH); Menne, Andreas, 88079 Meersburg (DE)
(74) Vertreter: Gauger, Hans-Peter, Dipl.-Ing.

(56) Entgegenhaltungen:
- EP-A- 0 317 507
- EP-A- 0 421 285
- DE-A- 4 414 903
- US-A- 3 830 240
- US-A- 4 823 793
- US-A- 5 387 190

## Beschreibung

Die Erfindung bezieht sich auf eine flexible Metallsonde, die als ein Wellenleiter in das Lumen eines bei der intrakorporalen Stoßwellen-Lithotripsie verwendeten Endoskops einführbar ist.

Bei der intrakorporalen Stoßwellen-Lithotripsie wird durch eine Metallsonde eine Stoßwelle übertragen, die an dem proximalen Ende der Sonde durch eine dort zur Übertragung kommende Stoßenergie erzeugt wird. Diese Stoßenergie wird bspw. bei einem aus der EP 0 317 507 B1 bekannten Lithotripter durch ein pneumatisch angetriebenes Schlagteil erzeugt, das in einem umgebenden Führungsrohr auf eine hohe Geschwindigkeit beschleunigt wird, um für die Dauer eines extrem kurzen Aufpralls gegen eine mit einem Sondenkopf vergrößerten Querschnitts gebildete Eingangsgrenzfläche der Sonde eine große Energiemenge an die Sonde zu übertragen. Durch eine aus dem Schlagimpuls resultierende Stoßenergie wird daher eine die Sonde durchlaufende Stoßwelle gebildet, die an der Sondenspitze für eine mittels des verwendeten Endoskops intrakorporal durchgeführte Zertrümmerung von Körpersteinen, wie Nieren-, Harnleiteroder Blasensteinen, genutzt wird.

Die Effizienz solcher Metallsonden ist abhängig von der Energieübertragung und auch der Energiewandlung, mit welcher somit die an den Sondenkopf übertragene Stoßenergie die Übermittlung an die Sondenspitze in der Ausbildung einer entstehenden Stoßwelle erfährt. Die Stoßwelle stellt sich dabei als eine Folge von sich wiederholenden Kompressionen und Expansionen dar. Mit der Wellenausbreitung ist auch eine Translationsbewegung der Sondenspitze verbunden, die final eine die Steinzertrümmerung auslösende Deformationswelle erzeugt. Es ist daher erklärbar, daß die geometrischen Abmessungen der Sonde die Ausbreitung der Stoßwelle stark beeinflussen. Deshalb ist auch eine Optimierung dieser geometrischen Abmessungen der Sonde eine angestrebte Zielsetzung, um für einen mit Ultraschallfrequenzen der Stoßwelle arbeitenden Lithotripter eine spontan abgangsfähige oder auch über einen separaten Ausspülkanal des verwendeten Endoskops direkt ausspülbare Partikelgröße eines mit der Sondenspitze zertrümmerten Körpersteins zu erhalten.

Die bisher bei den intrakorporalen Stoßwellen-Lithotriptern verwendeten Metallsonden sind für eine Anpassung an das Lumen der bei der Lithotripsie üblicherweise verwendeten Endoskope mit einem einheitlichen Durchmesser von 0.6 mm als einem Minimalwert und 3.2 mm als einem Maximalwert bei einer Sondenlänge von durchschnittlich 500 mm ausgeführt. Die Sonden mit dem kleineren Durchmesser sind dabei auch zur Verwendung bei sog. flexiblen Endoskopen geeignet, die auch mit einer steuerbaren Endoskopspitze versehen sein können und dabei eine größere Nutzlänge von bis zu 700 mm oder mehr aufweisen, um eine Biegung dieser flexiblen Endoskope in zwei Richtungen über ein Bogenmaß von bis zu 170° zu erlauben. Um für diese Biegung eine korrespondierend hohe Flexibilität der Sonde zu erhalten, ist es aus der US 5 449 363 bekannt, die Sonde längs der die Biegung des Endoskops aufnehmenden Teillänge mit einer Abflachung zu versehen, mit welcher dabei gleichzeitig ein unerwünschter Reibungskontakt der Sonde mit der umgebenden Wand des Lumens des Endoskops vermieden und die zusätzliche Möglichkeit geschaffen wird, diese Abflachung an vorbestimmten Stellen durch eine Bearbeitung mittels eines Lasers oder auch mittels eines elektrischen Lichtbogens mit Schlitzen zu versehen, um damit eine noch größere Flexibilität für die Sonde zu erhalten.

Als Folge ihrer relativ hohen Belastung mit der an den Sondenkopf zur Übertragung kommenden Stoßenergie unterliegen solche Sonden einer erhöhten Bruchgefahr, die sich über die Länge der Sonde an willkürlichen Positionen einstellen kann. Damit besteht bei der Endoskopie die Gefahr von ungewollten Verletzungen der Patienten und daneben auch die Gefahr von Beschädigungen der Wand des Arbeitskanals des Endoskops. Auch besteht die Möglichkeit, daß der Anwender mit einer unbeachtet gebliebenen schadhaften Sonde weiterarbeitet und daher seine Operationstätigkeit unvollkommen wird.

Aus der US-A-5 387 190 ist ein intrakorporaler Lithotripter bekannt, bei welchem eine als ein Wellenleiter dienende Metallsonde durch einen elektrisch angesteuerten Ultraschallwandler zu longitudinalen Schwingungen angeregt wird. Der Ultraschallwandler ist dafür mit piezokeramischen Scheiben ausgebildet mit einer Anordnung zwischen einem Reflektor und einem die Metallsonde tragenden Horn, sodass bei einer elektrischen Ansteuerung der piezokeramischen Scheiben über die Metallsonde Ultraschallwellen an deren distales Ende angeliefert werden, um dort eine Zertrümmerung von Körpersteinen zu bewirken.

Aus der DE-A-4 414 903 ist ein chirurgisches Instrument bekannt, bei welchem für einen Drehantrieb eines Werkzeuges mit einer Anordnung an dem vorderen Ende einer stab- oder rohrförmigen Antriebswelle eine Drehmomentbegrenzung beabsichtigt ist. Es ist dafür eine Sollbruchstelle mit Vorsprüngen an einem Verbindungsteil mit der Antriebswelle des Drehantriebes vorgesehen, sodass bei Überschreitung eines vorbestimmten Drehmoments durch ein Abbrechen von Vorsprüngen an dem Verbindungsteil der Antrieb hin zu dem Werkzeug dauerhaft unterbrochen wird.

Es besteht die Aufgabe der Bereitstellung einer für die Stoßwellen-Lithotripsie geeigneten Sonde, die ein sicheres Arbeiten zu garantieren vermag und die generell optimierte Verhältnisse für die Übermittlung der Stoßwelle an die Sondenspitze erwarten läßt.

Diese Aufgabe wird gemäß der vorliegenden Erfindung bei einer Metallsonde der durch den Oberbegriff des Patentanspruches 1 angegebenen Ausbildung gelöst mit den Merkmalen, die im Kennzeichnenden Teil des Anspruchs angegeben sind.

Die Realisierung einer Sollbruchstelle für die anfängliche Teillänge der Sonde ergibt eine optimale Einkreisung von möglichen Fehlerorten entlang der Sonde, die ihre Ursache in der sich ständig wiederholenden Schlageinwirkung des Projektils gegen das proximale Ende der Sonde haben können, sowie auch in einer ungleichförmigen Fortpflanzung der daraus resultierenden Stoßenergie hin zu dem distalen Ende der Sonde. Die Sollbruchstelle ist deshalb wie angegeben mit den bevorzugten Merkmalen der Ansprüche 13 bis 17 so ausgebildet, daß sie die weiteren Merkmale der erfindungsgemäßen Sonde, welche der Schaffung von optimierten Verhältnissen bei der Übermittlung der Stoßenergie an die Sondenspitze dienlich sind, unbeeinflusst läßt.

Durch die Beibehaltung des Nenndurchmessers an dem Ort der Sollbruchstelle wird nämlich primär an dem für die Aufnahme einer Stoßenergie vorgesehenen Sondenkopf die Voraussetzung dafür geschaffen, daß in Abstimmung auf die Größe dieses Nenndurchmessers ein Maximalwert der an dem Sondenkopf übernommenen Stoßenergie in die Sonde eingeleitet wird.
Es wird so die maßgebliche Voraussetzung dafür geschaffen, daß mit dieser maximalen Stoßenergie auch die Ausbildung der entstehenden Stoßwellen über die Hauptlänge der Sonde optimiert wird.

Eine Optimierung der entstehenden Stoßwelle wird weiter dadurch begünstigt, daß entlang der anfänglichen Teillänge der Sonde durch die Beibehaltung des Nenndurchmessers die bei der Ausbreitung der Stoßwellen auftretenden Transportverluste der Stoßenergie minimiert werden. Diese anfängliche Teillänge der Sonde, die sich an einem in aller Regel vorhandenen Querschnittssprung unmittelbar an den Sondenkopf anschließt, sollte daher auch eine Bemessung erhalten, welche die Restlänge der Sonde bis hin zu der Sondenspitze auf Bemessungswerte reduzieren läßt, mit denen vorrangig das flexible Verhalten der Sonde ohne jede nachteilige Beeinflussung des weiteren Stoßwellenverlaufs optimiert werden kann.

Eine weitere Optimierung kann damit erhalten werden, daß bei der erfindungsgemäßen Sonde eine definierte mittlere Teillänge eine stetigen Verkleinerung von dem Nenndurchmesser auf einen kleineren Sondendurchmesser unmittelbar im Anschluß an die den Nenndurchmesser aufweisende anfängliche Teillänge der Sonde aufweist. Die stetige Verkleinerung des Durchmessers sollte dabei optimal einen Kurvenverlauf gemäß einer Exponentialfunktion erhalten, um für die Stoßwellen größere Verluste der Stoßenergie zu vermeiden. Der kleinere Sondendurchmesser wird dabei im Verhältnis zu dem Nenndurchmesser der Sonde zweckmäßig mit der Vorgabe bestimmt, wie sich die Flexibilität der Sonde in den Grenzen optimieren läßt, die bspw. für eine Verwendungsmöglichkeit bei einem flexiblen Endoskop vorausgesetzt werden müssen. Gleichzeitig muß dieser kleinere Sondendurchmesser in Abstimmung mit einer noch anschließenden Teillänge der Sonde, über welche der kleinere Sondendurchmesser konstant beibehalten wird, auch so bemessen sein, daß für die finale Weiterleitung der Stoßwellen hin zu der im wesentlichen wieder den Nenndurchmesser aufweisenden Sondenspitze wiederholt unnötige Übergangsverluste der transportierten Stoßenergie vermieden werden. Der Übergang zu der Sondenspitze sollte daher ebenfalls mit einem stetigen Kurvenverlauf gemäß einer Exponentialfunktion ausgebildet werden mit der Vorgabe, daß der Nenndurchmesser der Sondenspitze im wesentlichen nur mit einer an der Sondenspitze vorgesehenen Stirnfläche ausgebildet wird. Die Sondenspitze erhält dann bei einer solchen speziellen Ausbildung eine Formgebung, die beim Einführen der Sonde in das Lumen eines Endoskops eine zusätzliche Führungsfunktion für die Sonde übernimmt. Auch wird dadurch erreicht, daß die Sondenspitze mit ihrer an der Stirnfläche positionierten größten Querschnittsfläche zur Anlage an dem jeweils zu zertrümmernden Körperstein kommen kann. Daraus ergibt sich eine Optimierung der Deformationswelie, die einen mit der Sondenspitze berührten Körperstein auf eine Partikelgröße zertrümmern läßt, welche ein problemloses Abführen ohne schädigende Einflüsse auf das umgebende Körpergewebe erwarten läßt.

Eine bestimmte Bemessung der einzelnen Teillängen der Sonde kann unter Berücksichtigung der vorstehend gegebenen Hinweise experimentell ermittelt werden, um bei einem durch das Lumen eines Endoskops vorgegebenen Nenndurchmesser der Sonde eine optimale Weiterleitung der Stoßwelle zwischen dem Sondenkopf und der Sondenspitze zu erhalten. Wichtig erscheinen dabei nur die Hinweise, daß die anfängliche Teillänge mit dem Nenndurchmesser der Sonde so groß wie möglich bemessen sein sollte, damit ein größtmöglicher Anteil der aus den einwirkenden Schlagimpulsen des Projektils resultiernden Stoßenergie in die Sonde eingeleitet wird und ein an dem Sondenkopf hinzunehmender Energieverlust nicht durch nachfolgende Transportverluste unnötig vergrößert wird. Weiterhin ist auch wichtig, daß eine Veränderung des Durchmessers längs der mittleren Teillänge der Sonde möglichst einer Exponentialkurve folgt, um so an den Durchmesserübergängen mögliche Veränderungen des Verlaufs der Stoßwellen für eine Optimierung der Steinzertrümmerung günstig zu beeinflussen. Die Bezugnahme auf eine definierte mittlere Teillänge soll weiterhin lediglich eine Ausbildung längs eines Abschnittes der Sonde beinhalten, der zwischen den beiden Teillängen der Sonde mit dem konstant beibehaltenen Nenndurchmesser und dem ebenfalls konstant beibehaltenen kleineren Sondendurchmesser verläuft. Dabei wäre die Vorgabe einzuhalten, daß die finale Teillänge der Sonde hin zu der Sondenspitze noch eine genügende Konzentration der Stoßenergie für die anschliessende Steinzertrümmerung erreichen lassen muß.

Ein Ausführungsbeispiel der Erfindung ist in der Zeichnung schematisch dargestellt und wird nachfolgend näher erläutert. Es zeigen
- Fig. 1: eine Schemadarstellung einer erfindungsgemäß ausgebildeten Metallsonde und
- Fig. 2: eine Schnittdarstellung des Sondenkopfes und einer zur Befestigung der Metallsonde an dem Handstück eines Lithotripters vorgesehenen Schraubkappe.

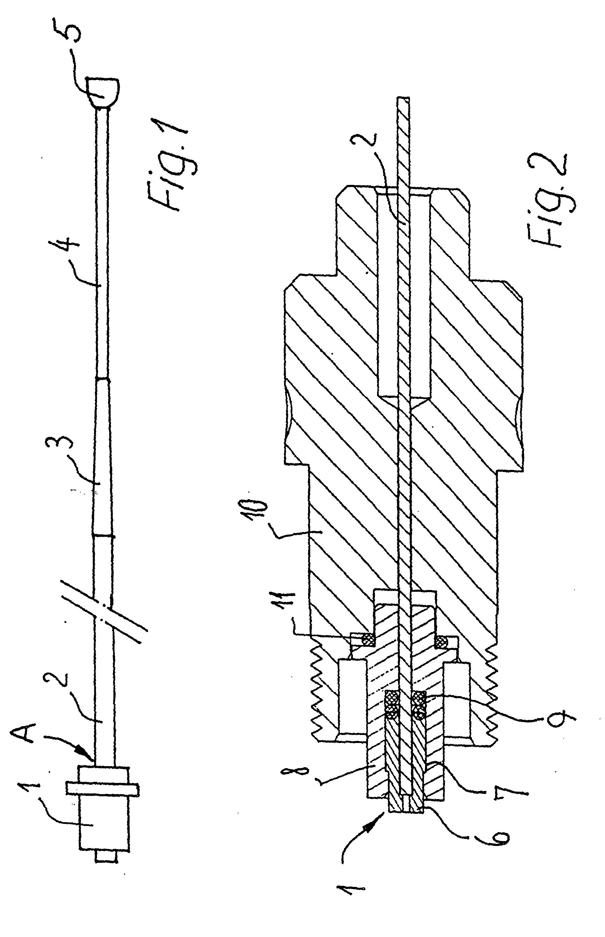

Eine gemäß der Erfindung ausgeführte Metallsonde zur Verwendung bei der intrakorporalen Stoßwellen-Lithotripsie ist gemäß der Darstellung in Fig. 1 der Zeichnung mit einem Sondenkopf 1 ausgebildet, der einen Querschnitt größer als ein vorbestimmter Nenndurchmesser der Sonde aufweist, mit welchem die Sonde an das Lumen eines für eine intrakorporale Stoßwellen-Lithotripsie verwendeten Endoskops angepaßt ist.

Der Sondenkopf 1 ist so bemessen, daß die Sonde mittels einer Schraubkappe an dem Handstück eines Lithotripters bspw. einer Ausbildung gemäß der EP 0 317 507 gehalten werden kann und dabei zusätzlich noch die Möglichkeit für die Anordnung einer Dichtungsmanschette besteht. Eine solche Dichtungsmanschette soll mit ihren eigenelastischen Materialeigenschaften als ein Dämpfer wirken und soll daneben eine Beeinflussung der Stoßwelle ergeben, die für eine Weiterleitung durch die Sonde entsteht, sobald ein bei diesem bekannten Lithotripter pneumatisch angetriebenes Schlagteil oder Projektil den Sondenkopf beaufschlagt und durch den damit erhaltenen Schlagimpuls eine Stoßenergie erzeugt wird. Anstelle eines pneumatischen Antriebs kann für das Schlagteil auch ein hydraulischer oder ein elektromagnetischer Antrieb realisiert sein.

Der an das Lumen eines Endoskops angepaßte Nenndurchmesser der Sonde wird für eine anfängliche Teillänge 2 eingehalten, die sich über einen Querschnittssprung unmittelbar an den Sondenkopf 1 anschließt. Diese den Nenndurchmesser aufweisende anfängliche Teillänge 2 schafft die Voraussetzung dafür, daß eine aus der übergebenen Stoßenergie entstehende Stoßwelle mit einem möglichst geringen Transportverlust weitergeleitet wird an eine mittlere Teillänge 3 der Sonde, entlang welcher der Nenndurchmesser stetig und vorzugsweise mit einem Kurvenverlauf gemäß einer Exponentialfunktion auf einen kleineren Sondendurchmesser verkleinert wird. Der kleinere Sondendurchmesser wird für eine anschließende weitere Teillänge 4 der Sondenspitze konstant beibehalten und ist dabei so bemessen, daß mit dieser weiteren Teillänge 4 in der Kombination mit der mittleren Teillänge 3 eine Flexibilität erhalten wird, welche die Sonde auch problemlos in das Lumen eines flexiblen Endoskops einführen läßt, das einer Biegung mit einer Umlenkung von bspw. bis zu 170° folgen kann. Die den kleineren Sondendurchmesser aufweisende Teillänge 4 der Sonde wird daher vorzugsweise für die Verwendung bei einem flexiblen Endoskop in Abhängigkeit von dem Ausmaß seiner Biegung bemessen, die für ein Arbeiten bspw. mittels einer aktiv steuerbaren Endoskopspitze angestrebt wird.

Die Sonde ist dann noch mit einer Sondenspitze 5 versehen, die sich an die weitere Teillänge 4 unmittelbar anschließt. Die Sondenspitze ist mit einer Stirnfläche versehen, die wieder im wesentlichen den Nenndurchmesser aufweist. Der kleinere Sondendurchmesser ist dabei vorzugsweise ebenfalls entsprechend dem Kurvenverlauf einer Exponentialfunktion hin zu der Stirnfläche der Sondenspitze vergrößert. Die für diese finale Teillänge der Sonde vorgegebene Exponentialfunktion weicht an von der Exponentialfunktion, die den Kurvenverlauf der mittleren Teillänge 3 der Sonde bestimmt.

Die erfindungsgemäße Metallsonde oder Teillängen der Sonde bestehen zweckmäßig aus einer Nickel-Titan-Legierung oder aus Edelstahl bzw. auch aus einer Kombination dieser Materialien. Die verschiedenen Teillängen der Sonde können dabei mit unterschiedlichen Materialeigenschaften versehen sein. Eine gegenüber der Restlänge der Sonde vergrößerte Steifheit kann so bspw. durch eine Temperaturbehandlung für die anfängliche Teillänge der Sonde vorgegeben sein, womit die Restlänge der Sonde dann eine zur Verwendung bei einem flexiblen Endoskop erwünschte höhere Flexibilität erhält. Auch kann daran gedacht werden, die gesamte Sonde oder Teillängen der Sonde bleibend vorzubiegen, um für das Arbeiten mit einem flexiblen Endoskop den größeren Stellkräften entgegenzuwirken, die sich bei der Steuerung eines größeren Bewegungsradius des Endoskops einstellen.

Eine materialbedingte Beeinflussung der Sonde wird auch für die Ausbildung einer Sollbruchstelle zu dem Zweck vorgenommen, eine sich während des Arbeitens mit der Sonde einstellende Materialermüdung als Folge einer vielfach wiederholten Schlageinwirkung gezielt einzukreisen mit einer Konzentration auf eine vorbestimmte Stelle, die keine Gefahr für den Patienten während der Steinzertrümmerung ergibt. Eine solche Sollbruchstelle auf welche in Fig. 1 mit dem Pfeil A hingewisen ist, wird zweckmäßig in der Nähe des Überganges des Sondenkopfes 1 zu der den Nenndurchmesser aufweisenden anfänglichen Teillänge 2 der Sonde vorgesehen. Die Sollbruchstelle kann mit einem örtlich verringerten Querschnitt der Sonde bspw. in der Ausbildung eine Kerbe oder auch durch eine örtlich abweichende Materialbehandlung erhalten werden, ohne daß dafür die Abmessung der Sonde an der betreffenden Schwachstelle verändert wird. Damit kann eine nachteilige Beeinflussung der Wellenausbreitung vermieden werden, wie es sonst bei der Ausbildung einer Kerbe der Fall sein kann.

Eine weitere Möglichkeit für die Vorgabe einer Sollbruchstelle wäre eine Verklebung des Sondenkopfes mit der anfänglichen Teillänge der Sonde oder auch das Vorsehen einer Quetschverbindung an dieser Stelle. Auch könnte der Sondenkopf mit einer etwas geringeren Festigkeit als die Restlänge der Sonde ausgebildet werden, um bspw. nach einer vorbestimmten Anzahl von Schlageinwirkungen des Pojektils gegen das proximale Ende der Sonde eine Zerstörung des Sondenkopfes zuzulassen.

Eine abweichende und lokal begrenzte Materialbehandlung kann daneben auch bspw. für die den Nenndurchmesser aufweisende anfängliche Teillänge der Sonde an einer Stelle vorgesehen sein, für welche weniger die Ausbildung einer Sollbruchstelle als vielmehr eine erhöhte Flexibilität unter Beibehaltung des Nenndurchmessers interessiert. Die Sonde könnte dann auch über einen seitlich vorgesehenen Eintritt in den Arbeitskanal eines Endoskops problemloser eingeführt werden.

Abschließend wird zu der Darstellung in Fig. 2 noch darauf hingewiesen, daß es für die Übertragung der Stoßenergie an die Sonde und die Übermittlung der daraus entstehenden Stoßwellen an die Sondenspitze auch von Vorteil sein kann, wenn an dem proximalen Ende der Sonde bestimmte Vorkehrungen für eine axiale Führung der Sonde getroffen werden. Der Sondenkopf kann daher zweckmäßig mit einem Metallkopf 6 ausgebildet sein, der auf die Sonde aufgesteckt oder sonstwie an der Sonde befestigt ist. Der Metallkopf 6 ist in eine Führungsbohrung 7 einer umgebenden Führungshülse 8 eingesetzt, wobei die Führungshülse das proximale Ende der Sonde in einer axialen Fortsetzung der Führungsbohrung 7 axial führt.

In der Führungsbohrung 7 sind auch zwei O-Ringe 9 aufgenommen, die auf die Sonde aufgesteckt sind und sich zwischen dem Boden der Führungsbohrung 7 und dem Metallkopf 6 abstützen, um damit die Sonde in axialer Richtung entgegen den Schlagimpulsen vorzuspannen, die auf den Metallkopf 6 durch ein bspw. pneumatisch angetriebenes Schlagteil eines Lithotripters einwirken. An einem Handstück des Lithotripters kann die Sonde mittels einer Schraubkappe 10 unter Zwischenfügung der Führungshülse 8 sowie eines weiteren O-Ringes 11 befestigt sein. Für eine alternative Ausbildung kann jedoch auch daran gedacht sein, daß die Führungshülse 8 eine einstückige Ausbildung mit der Schraubkappe 10 erhält, jedoch ist die hier gezeigte getrennte Ausbildung unter dem Gesichtspunkt zu bevorzugen, daß die Führungshülse in diesem Fall aus einem flexiblen Material bestehen kann, mit welchem die Rückstellkräfte ergänzt werden können, die mit den beiden O-Ringen 9 erhalten werden.

## Patentansprüche

1. Flexible Metallsonde, die als ein Wellenleiter in das Lumen eines bei der intrakorporalen Stoßwellen-Lithotripsie verwendeten Endoskops einführbar ist, wobei das proximale Ende der Sonde einen für die Aufnahme einer Stoßenergie vorgesehenen Sondenkopf (1) mit einem Querschnitt größer als ein Vorbestimmter Nenndurchmesser der Sonde aufweist und die das distale Ende der Sonde bildende Sondenspitze (5) für eine Zertrümmerung von Körpersteinen durch mit der Sonde vermittelte Stoßwellen genutzt wird,
**dadurch gekennzeichnet, daß** an dem Übergang des Sondenkopfes (1) zu der den Nenndurchmesser aufweisenden anfänglichen Teillänge (2) der Sonde oder in der Nähe dieses Überganges eine Sollbruchstelle (A) der Sonde ausgebildet ist.

2. Flexible Metallsonde nach Anspruch 1, **dadurch gekennzeichnet, daß** der Nenndurchmesser der anfänglichen Teillänge (2) der Sonde längs einer definierten mittleren Teillänge (3) auf einen kleineren Sondendurchmesser stetig verkleinert ist, wobei dieser kleinere Sondendurchmesser über eine sich unmittelbar anschließende weitere Teillänge (4) konstant beibehalten und in der Nähe der Sondenspitze (5) wieder im wesentlichen auf einen dem Nenndurchmesser der Sonde angenäherten größeren Durchmesser stetig vergrößert ist.

3. Flexible Metallsonde nach Anspruch 1, **dadurch gekennzeichnet, daß** die stetige Verkleinerung des Nenndurchmessers der Sonde längs ihrer mittleren Teillänge (3) mit einem Kurvenverlauf gemäß einer Exponentialfunktion ausgeführt ist.

4. Flexible Metallsonde nach Anspruch 3, **dadurch gekennzeichnet, daß** die stetige Vergrößerung des kleineren Sondendurchmessers im wesentlichen auf den Nenndurchmesser der Sondenspitze (5) mit einem Kurvenverlauf gemäß einer Exponentialfunktion ausgeführt ist, die abweicht von der Exponentialfunktion, die für die stetige Verkleinerung des Nenndurchmessers der Sonde längs ihrer mittleren Teillänge (3) vorgegeben ist.

5. Flexible Metallsonde nach einem der Ansprüche 1 bis 4, **dadurch gekennzeichnet, daß** der Nenndurchmesser der Sondenspitze (5) im wesentlichen nur für eine mit der Sondenspitze ausgebildete Stirnfläche vorgegeben ist.

6. Flexible Metallsonde nach einem der Ansprüche 2 bis 5, **dadurch gekennzeichnet, daß** die weitere Teillänge (4) der Sonde unmittelbar anschließt an die stetige Vergrößerung der Sondenspitze (5).

7. Flexible Metallsonde nach einem der Ansprüche 1 bis 6, **dadurch gekennzeichnet, daß** die Sonde oder Teillängen (1,2,3,4,5) der Sonde aus einer Nickel-Titan-Legierung und/oder aus Edelstahl bestehen.

8. Flexible Metallsonde nach einem der Ansprüche 2 bis 7, **dadurch gekennzeichnet, daß** die verschiedenen Teillängen (1,2,3,4,5) der Sonde aus Materialien mit unterschiedlichen Eigenschaften und/oder aus Materialien bestehen, die unterschiedlich wärmebehandelt sind.

9. Flexible Metallsonde nach einem der Ansprüche 1 bis 8, **dadurch gekennzeichnet, daß** die anfängliche Teillänge (2) der Sonde mit einer durch eine Temperaturbehandlung erreichten größeren Steifheit versehen ist als die Restlänge der Sonde.

10. Flexible Metallsonde nach einem der Ansprüche 1 bis 9, **dadurch gekennzeichnet, daß** die gesamte Sonde oder Teillängen (2,3,4,5) der Sonde im Anschluß an den Sondenkopf (1) bleibend vorgebogen sind.

11. Flexible Metallsonde nach einem der Ansprüche 1 bis 10, **dadurch gekennzeichnet, daß** der Sondenkopf (1) mit einem an der Sonde befestigten Metallkopf (6) ausgebildet ist, der in eine axiale Führungsbohrung (7) einer umgebenden Führungshülse (8) für das proximale Ende der Sonde eingesetzt und durch wenigstens einen auf die Sonde aufgeschobenen und in der Führungsbohrung (7) mit einer leichten Vorspannung aufgenommenen O-Ring (9) axial vorgespannt ist.

12. Flexible Metallsonde nach Anspruch 11, **dadurch gekennzeichnet, daß** die Führungshülse (8) aus einem flexiblen Material besteht und für eine Befestigung der Sonde an einem Handstück eines Lithotripters mittels einer mit dem Handstück verschraubbaren Schraubkappe (10) angepasst ist.

13. Flexible Metallsonde nach einem der Ansprüche 1 bis 12, **dadurch gekennzeichnet, daß** die Sollbruchstelle (A) mit einer Wärmebehandlung der Sonde vorgegeben ist, welche die Festigkeit der Sonde in der Nähe des Überganges zu der anfänglichen Teillänge (2) der Sonde örtlich schwächt.

14. Flexible Sonde nach einem der Ansprüche 1 bis 13, **dadurch gekennzeichnet, daß** die Sollbruchstelle (A) mit einem im Vergleich zu dem Nenndurchmesser der anfänglichen Teillänge (2) der Sonde örtlich verringerten Querschnitt ausgeführt wird.

15. Flexible Sonde nach Anspruch 14, **dadurch gekennzeichnet, daß** die Sollbruchstelle (A) als eine Kerbe ausgeführt wird.

16. Flexible Sonde nach einem der Ansprüche 1 bis 15, **dadurch gekennzeichnet, daß** die Sollbruchstelle (A) durch eine Klebeverbindung des Sondenkopfes (1) mit der anfänglichen Teillänge (2) der Sonde vorgegeben ist.

17. Flexible Sonde nach einen der Ansprüche 1 bis 16, dadurch gekennzeichet, daß die Sollbruchstelle (A) durch eine Quetschverbindung des Sondenkopfes (1) mit der anfänglichen Teillänge (2) der Sonde vorgegeben ist.

## Claims

1. A metallic flexible probe which as a wave guide is adapted for being inserted into the lumen of an endoscope for use in the intracorporeal shock wave lithotripsy whereby the proximal end of the probe comprises a probe head (1) provided for taking up an impact energy and having a cross-section which is larger than a predetermined nominal diameter of the probe and whereby the tip portion (5) of the probe forming the distal end of the probe is used for a fragmentation of calculi by means of shock waves that are transmitted by the probe,
**characterised in that** a predetermined breaking point (A) is provided at the transition of the probe head (1) to the initial partial length (2) of the probe which is provided with the nominal diameter or in the proximity of this transition.

2. The metallic flexible probe according to claim 1, **characterised in that** the nominal diameter of the initial partial length (2) of the probe is continuously reduced to a smaller diameter of the probe along a definite intermediate partial length (3) whereby this smaller diameter of the probe is retained constant over an immediately adjoining additional partial length (4) and is continuously enlarged in the proximity of the tip portion (5) of the probe substantially to a larger diameter which substantially approximates the nominal diameter of the probe.

3. The metallic flexible probe according to claim 1, **characterised in that** the continuous reduction of the nominal diameter of the probe along its intermediate partial length (3) is provided with a curvature in accordance with an exponential function.

4. The metallic flexible probe according to claim 3, **characterised in that** the continuous enlargement of the smaller diameter of the probe substantially to the nominal diameter of the tip portion (5) of the probe is provided with a curvature according to an exponential function which is different from the exponential function which is predetermined for the continuous reduction of the nominal diameter of the probe along its intermediate partial length (3).

5. The metallic flexible probe according to any of the claims 1 to 4, **characterised in that** the nominal diameter of the tip portion (5) of the probe is substantially provided only for a front face which is formed by the tip portion of the probe.

6. The metallic flexible probe according to any of the claims 2 to 5, **characterised in that** the additional partial length (4) of the probe is immediately next to the continuous enlargement of the tip portion (5) of the probe.

7. The metallic flexible probe according to any of the claims 1 to 6, **characterised in that** the probe or partial lengths (1, 2, 3, 4, 5) of the probe consist of a nickel-titanium-alloy and/ or of stainless steel.

8. The metallic flexible probe according to any of the claims 2 to 7, **characterised in that** the different partial lengths (1, 2, 3, 4, 5) of the probe consist of materials of different properties and/or of materials of a different heat treatment.

9. The metallic flexible probe according to any of the claims 1 to 8, **characterised in that** the initial partial length (2) of the probe is provided with a larger stiffness as obtained by a heat treatment than the remaining length of the probe.

10. The metallic flexible probe according to any of the claims 1 to 9, **characterised in that** the entire probe or partial lengths (2, 3, 4, 5) of the probe next to the probe head (1) are permanently prebent.

11. The metallic flexible probe according to any of the claims 1 to 10, **characterised in that** the probe head (1) is formed with a metallic head (6) which is fixed on the probe and which is inserted into an axial guide bore (7) of a surrounding guide bush (8) for the proximal end of the probe and axially pre stressed by means of at least one O-ring (9) which is slipped-on over the probe and retained within the guide bore (7) with a slight prestress.

12. The metallic flexible probe according to claim 11, **characterised in that** the guide bush (8) consists of a flexible material and is adapted for enabling a fixation of the probe on a handpiece of a lithotripter by means of a screw cap (10) which is adapted for being screw connected with the handpiece.

13. The metallic flexible probe according to any of the claims 1 to 12, **characterised in that** the predetermined breaking point (A) is predetermined by a heat treatment for the probe which locally reduces the strength of the probe in the vicinity of the transition to the initial partial length (2) of the probe.

14. The metallic flexible probe according to any of the claims 1 to 13, **characterised in that** the predetermined breaking point (A) comprises a locally reduced cross-section in comparison with the initial partial length (2) of the probe which is provided with the nominal diameter.

15. The metallic flexible probe according to claim 14, **characterised in that** the predetermined breaking point (A) is formed as a notch.

16. The metallic flexible probe according to any of the claims 1 to 15, **characterised in that** the predetermined breaking point (A) is predetermined by an adhesive connection of the probe head (1) with the initial partial length (2) of the probe.

17. The metallic flexible probe according to any of the claims 1 to 16, **characterised in that** the predetermined breaking point (A) is predetermined by a crimp connection of the probe head (1) with the initial partial length (2) of the probe.

## Revendications

1. Sonde métallique flexible apte à être introduit en tant que guide d'ondes dans le lumen d'un endoscope utilisé pour la lithotripsie intracorporel à ondes de choc, dans laquelle l'extrémité proximale de la sonde comprend une tête de sonde (1) disposée pour la réception d'une énergie de choc, à un profil en travers plus grand qu'un diamètre nominal prédéterminé de la sonde, pendant que la pointe de sonde (5), qui constitue l'extrémité distale de la sonde, est utilisée pour la fragmentation des calculs dans le corps moyennant des ondes de choc transférées par la sonde,
**caractérisée en ce qu'**à la transition de ladite tête de sonde (1) vers le tronçon partiel initial (2), qui présente le diamètre nominal, de la sonde, ou à la proximité de cette transition, un point destiné à la rupture (A) de la sonde est formé.

2. Sonde métallique flexible selon la revendication 1, **caractérisée en ce que** le diamètre nominal dudit tronçon partiel initial (2) de la sonde est réduit, en continu, à un plus petit diamètre de sonde le long d'un tronçon partiel central défini (3), ce plus petit diamètre de sonde étant maintenu à une valeur constante le long d'un autre tronçon partiel directement suivant (4) et étant élargi, en continu, encore à un plus grand diamètre essentiellement approximé au diamètre nominal de la sonde, à la proximité de ladite pointe de sonde (5).

3. Sonde métallique flexible selon la revendication 1, **caractérisé en ce que** le réduction continue du diamètre nominal de la sonde est réalisée le long de son tronçon partiel central (3) à une allure de courbe selon une fonction exponentielle.

4. Sonde métallique flexible selon la revendication 3, **caractérisée en ce que** l'élargissement continu du plus petit diamètre de la sonde est réalisé essentiellement au diamètre nominal de ladite pointe de sonde (5) à une allure de courbe selon une fonction exponentielle, qui varie de la fonction exponentielle prédéterminée pour la réduction continue du diamètre nominal de la sonde le long de son tronçon partiel central (3).

5. Sonde métallique flexible selon une quelconque des revendications 1 à 4, **caractérisée en ce que** le diamètre nominal de ladite pointe de sonde (5) n'est essentiellement prédéterminé que pour une face configurée à la pointe de la sonde.

6. Sonde métallique flexible selon une quelconque des revendications 2 à 5, **caractérisée en ce que** ledit autre tronçon (4) de la sonde est directement contiguë à l'élargissement continu de ladite pointe de sonde (5).

7. Sonde métallique flexible selon une quelconque des revendications 1 à 6, **caractérisée en ce que** la sonde ou des tronçons partiels (1, 2, 3, 4, 5) de la sonde sont fabriqués en un alliage de nickel/titane et/ou en acier surfin.

8. Sonde métallique flexible selon une quelconque des revendications 2 à 7, **caractérisée en ce que** les divers tronçons partiels (1, 2, 3, 4, 5) de la sonde consistent en matériaux aux caractéristiques différentes et/ou en matériaux traités par des processus thermiques différents.

9. Sonde métallique flexible selon une quelconque des revendications 1 à 8, **caractérisée en ce que** ledit tronçon partiel initial (2) de la sonde est donné une rigidité, qui est achevée par un traitement thermique et qui est plus grande que la rigidité de la longueur résiduelle de la sonde.

10. Sonde métallique flexible selon une quelconque des revendications 1 à 9, **caractérisée en ce que** l'entière sonde ou des tronçons partiels (2, 3, 4, 5) de la sonde présentent un pré-cintrage permanent dans la zone jointe à ladite tête de sonde (1).

11. Sonde métallique flexible selon une quelconque des revendications 1 à 10, **caractérisée en ce que** ladite tête de sonde (1) est configurée à une tête métallique (6) fixée à la sonde, qui est introduit dans un alésage de guidage axial (7) d'une douille de guidage (8) l'entourant pour l'extrémité proximale de la sonde et qui est précontraint en sens axial par au moins un anneau torique (9) poussé sur la sonde et reçu dans ledit alésage de guidage (7) à une faible précontrainte.

12. Sonde métallique flexible selon la revendication 11, **caractérisée en ce que** ladite douille de guidage (8) consiste en un matériau flexible et est adaptée, moyennant un bouchon fileté (10) apte à être vissé à une pièce à main, pour l'attache de la sonde à une pièce à main d'un lithotripteur.

13. Sonde métallique flexible selon une quelconque des revendications 1 à 12, **caractérisée en ce que** ledit point destiné à la rupture (A) est défini par un traitement thermique de la sonde, qui affaiblit localement la résistance mécanique à la proximité de la transition vers le tronçon partiel initial (2) de la sonde.

14. Sonde flexible selon une quelconque des revendications 1 à 13, **caractérisée en ce que** ledit point destiné à la rupture (A) est réalisé par un profil en travers localement réduit, qui est plus petit que le diamètre nominal dudit tronçon partiel initial (2) de la sonde.

15. Sonde flexible selon la revendication 14, **caractérisée en ce que** ledit point destiné à la rupture (A) est réalisé sous forme d'une encoche.

16. Sonde flexible selon une quelconque des revendications 1 à 15, **caractérisée en ce que** ledit point destiné à la rupture (A) est prédéterminé par un assemblage collé de ladite tête de sonde (1) audit tronçon partiel initial (2) de la sonde.

17. Sonde flexible selon une quelconque des revendications 1 à 16, **caractérisée en ce que** ledit point destiné à la rupture (A) est prédéterminé par une connexion sertie de ladite tête de sonde (1) audit tronçon partiel initial (2) de la sonde.
